# EUROPEAN PATENT SPECIFICATION

(11) **EP 3 000 876 B1**
(45) Date of publication and mention of the grant of the patent: **24.04.2019**
(21) Application number: 14801845.0
(22) Date of filing: 14.05.2014
(51) Int. Cl.: C12N 5/0783, A61K 35/14, A61P 31/04, A61P 31/12, A61P 35/02, A61K 35/51, A61K 35/17, A61K 35/545

(54) **METHOD FOR PREPARING NK CELLS**
VERFAHREN ZUR HERSTELLUNG VON NK-ZELLEN
MÉTHODE DE PRÉPARATION DE CELLULES NK

(30) Priority: 22.05.2013 JP 2013107568
(43) Date of publication of application: 30.03.2016
(73) Proprietor: Kyushu University National University Corporation, Fukuoka-shi Fukuoka 812-8581 (JP); GAIA BioMedicine Inc., Chuo-ku Fukuoka-City Fukuoka 810-0023 (JP)
(72) Inventor: YONEMITSU, Yoshikazu, Fukuoka-shi Fukuoka 812-8581 (JP); SAITO, Satoru, Fukuoka-shi Fukuoka 812-8581 (JP); HARADA, Yui, Fukuoka-shi Fukuoka 812-8581 (JP); YAZAKI, Yuichiro, Tokyo 107-6017 (JP); ISHIDAO, Takefumi, Tokyo 107-6017 (JP)
(74) Representative: Arends, William Gerrit
(86) International application number: PCT/JP2014/002541
(87) International publication number: WO 2014/188680

(56) References cited:
- WO-A1-2007/032634
- WO-A1-2010/099539
- WO-A1-2011/080740
- WO-A1-2011/103882
- WO-A1-2011/103882
- WO-A1-2012/009422
- WO-A1-2012/009422
- AYELLO J ET AL: "Characterization of natural killer and natural killer-like T cells derived from ex vivo expanded and activated cord blood mononuclear cells: Implications for adoptive cellular immunotherapy", EXPERIMENTAL HEMATOLOGY, ELSEVIER INC, US, vol. 37, no. 10, 1 October 2009 (2009-10-01), pages 1216-1229, XP026612092, ISSN: 0301-472X, DOI: 10.1016/J.EXPHEM.2009.07.009 [retrieved on 2009-07-26]
- WILLIAMS N S ET AL: "GENERATION OF LYTIC NATURAL KILLER 1.1+, LY-49- CELLS FROM MULTIPOTENTIAL MURINE BONE MARROW PROGENITORS IN A STROMA-FREE CULTURE: DEFINITION OF CYTOKINE REQUIREMENTS AND DEVELOPMENTAL INTERMEDIATES", THE JOURNAL OF EXPERIMENTAL MEDICINE, ROCKEFELLER UNIVERSITY PRESS, US, vol. 186, no. 9, 3 November 1997 (1997-11-03), pages 1609-1614, XP000857808, ISSN: 0022-1007, DOI: 10.1084/JEM.186.9.1609
- LI YANG ET AL: "Expansion of CIK/NK cells from cord blood by using different combinations of stem cell factor, FLT3 ligand and interleukin 2, 7, 15 in vitro", ZHONGGUO SHIYAN XUEYEXUE ZAZHI - JOURNAL OF EXPERIMENTALHEMATOLOGY, ZHONGGUO SHIYAN XUEYEXUE ZAZHISHE, BEIJING, CN, vol. 12, no. 3, 1 June 2004 (2004-06-01), pages 350-354, XP009133162, ISSN: 1009-2137
- SATORU SAITO ET AL: "Ex Vivo Generation of Highly Purified and Activated Natural Killer Cells from Human Peripheral Blood", HUMAN GENE THERAPY METHODS, vol. 24, no. 4, 1 August 2013 (2013-08-01) , pages 241-252, XP055154762, ISSN: 1946-6536, DOI: 10.1089/hgtb.2012.183

## Description

### Technical Field

The present invention relates to a method for preparing natural killer cells (NK cells) having a high cytotoxic activity from hematopoietic precursor cells with high purity and a high expansion factor without using a serum or feeder cells of an animal, and a pharmaceutical composition containing the NK cells obtained by the method.

### Background Art

NK cells do not attack normal cells expressing MHC class I molecules but mainly attack cells in which the expression of the MHC class I molecules is reduced or lost. Since the expression of the MHC class I molecules is reduced in cancer cells or cells infected with a virus, NK cells can attack these cells. Therefore, if allogeneic NK cells are used in cell therapy of a cancer or an infectious disease, it is advantageous that there is no need to precedently immunize the NK cells for causing them to recognize target cells, and that an adverse reaction of GVH (Graft-versus-host) disease can be avoided. Actually, according to reports of Miller et al. (Non Patent Literature 1) and Rubnitz et al. (Non Patent Literature 2), when a cancer patient was a recipient and concentrated NK cells obtained from fresh peripheral blood mononuclear cells of a healthy donor closely related to the recipient were transplanted, the transplanted NK cells temporarily survived without causing an adverse reaction in the recipient and retained their cytotoxic activity. There is, however, no report on a clinical trial showing the effectiveness of NK cell transplantation therapy. One of the reasons is that the number of cells collectable from a donor by lymphocyte apheresis is limited, and hence, NK cells in number sufficient for killing target cells, such as cancer cells or cells infected with a pathogen, cannot be caused to stay in the body of a recipient until the target cells are killed. According to, for example, Non Patent Literature 2, a survival period of NK cells is not correlated with the number of administered NK cells, but is merely 2 to 189 days, with a median as small as 10 days. Therefore, in order to cause NK cells in number sufficient for killing target cells, such as cancer cells or cells infected with a pathogen, to stay in the body of a recipient until the target cells are killed, it is necessary to frequently repeat the NK cell transplantation, which is a great burden of the patient. Accordingly, a technique in which NK cells obtained from a donor are once cultured in a test tube to obtain NK cells in number sufficient for killing target cells has been developed. In this technique, use of a serum or feeder cells of an animal is not preferred because a risk of infection is otherwise caused in prepared NK cells.

Umbilical cord bloods of various blood types are classified and stored in an umbilical cord blood bank, and thus if an umbilical cord blood is used as an origin of NK cells for use in a treatment, it is easy to select, based on the blood type of a patient, an umbilical cord blood of a blood type that has high histocompatibility and low possibility of an adverse reaction caused by the transplantation. Therefore, a technique in which NK cells for use in a treatment are prepared from an umbilical cord blood without using a serum or feeder cells of an animal has recently attract attention. Spanholtz et al. (Non Patent Literature 3) reported that NK cells were prepared in number ten thousand times or more in 6 weeks from CD34-positive cells derived from a cryopreserved umbilical cord blood. It cannot be said, however, that the cytotoxic activity of the NK cells obtained by the method of this report is high. Besides, in the conventional technique, for the preparation of NK cells from hematopoietic precursor cells, it is necessary to replace, during the preparation, a medium with one having a different cytokine composition.

### Citation List

### Non Patent Literature

Non Patent Literature 1: Miller, J. S. et al., Blood, 105: 3051 (2005)
Non Patent Literature 2: Rubnitz, J. E. et al., J. Clin. Oncol., 28: 955 (2010)
Non Patent Literature 3: Spanholtz et al., PLos ONE, 5: e9221 (2010)

### Summary of Invention

### Technical Problem

Accordingly, it is necessary to develop a technique in which NK cells having a high cytotoxic activity can be prepared with high purity, without using a serum or feeder cells of an animal, from hematopoietic precursor cells including hematopoietic precursor cells derived from an umbilical cord blood or an adult blood cell tissue, and hematopoietic precursor cells prepared from induced pluripotent stem cells, embryonic stem cells, adult stem cells or the like. Besides, in order to simplify operation procedures, it is necessary to develop a simpler culturing condition for preparation of hematopoietic precursor cells. Furthermore, it is also necessary to develop a simpler culturing condition for preparing NK cells from the hematopoietic precursor cells.

### Solution to Problem

The present invention provides a method for preparing NK cells. The method for preparing NK cells of the present invention includes the steps of expanding hematopoietic precursor cells under a single culturing condition; and differentially inducing the cells obtained in the expanding step into NK cells.

In the method for preparing NK cells, a medium used in the step of expanding hematopoietic precursor cells under a single culturing condition may be supplemented with IL-15, SCF, IL-7 and Flt3L in some cases.

In the method for preparing NK cells, the medium used in the step of expanding hematopoietic precursor cells under a single culturing condition may be supplemented further with TPO in some cases.

In the method for preparing NK cells, the step of differentially inducing the NK cells may include culturing the expanded hematopoietic precursor cells under a culturing condition containing IL-2 in some cases.

In the method for preparing NK cells of the present invention, the medium used in each of the steps may be supplemented with a human AB-type serum and/or a human serum albumin in some cases.

In the method for preparing NK cells of the present invention, the hematopoietic precursor cells may be at least one of hematopoietic precursor cells selected from the group consisting of hematopoietic precursor cells contained in an umbilical cord blood and/or an adult blood cell tissue, hematopoietic precursor cells differentially induced from induced pluripotent stem cells, embryonic stem cells and/or adult stem cells, and hematopoietic precursor cells directly converted from differentiated cells in some cases.

In the method for preparing NK cells of the present invention, a major histocompatibility complex (MHC) or a killer immunoglobulin-like receptor (KIR) may not be the same between the hematopoietic precursor cells and a patient.

The present invention provides a pharmaceutical composition for use in cell therapy containing the NK cells prepared by the preparation method of the present invention. The pharmaceutical composition of the present invention may contain, in addition to the NK cells prepared by the preparation method of the present invention, an NK cell precursor, T cells, an NKT cells, and hematopoietic precursor cells in some cases. The present invention provides a method for preparing the pharmaceutical composition for use in cell therapy of the present invention.

The pharmaceutical composition of the present invention may be used for treating an infectious disease and/or a cancer in some cases.

The present invention provides cell therapy. The cell therapy of the present invention includes a step of expanding hematopoietic precursor cells under a single culturing condition; and a step of differentially inducing the cells obtained in the expanding step into NK cells. In the cell therapy, a medium used in the step of expanding hematopoietic precursor cells under a single culturing condition may be supplemented with IL-15, SCF, IL-7 and Flt3L in some cases. In the cell therapy, the medium used in the step of expanding hematopoietic precursor cells under a single culturing condition may be supplemented further with TPO in some cases. In the cell therapy, the step of differentially inducing the NK cells may include culturing the expanded hematopoietic precursor cells under a culturing condition containing IL-2 in some cases. In the cell therapy, the medium used in each of the steps may be supplemented with a human AB-type serum and/or a human serum albumin. In the cell therapy, the hematopoietic precursor cells may be at least one of hematopoietic precursor cells selected from the group consisting of hematopoietic precursor cells contained in an umbilical cord blood and/or an adult blood cell tissue, hematopoietic precursor cells differentiation induced from induced pluripotent stem cells, embryonic stem cells and/or adult stem cells, and hematopoietic precursor cells directly converted from differentiated cells. In the cell therapy, the step of transplanting the NK cells into a patient may be a step of transplanting the NK cells together with other cells such as T cells or NKT cells in some cases. The cell therapy of the present invention may be employed for treating and/or preventing an infectious disease and/or a cancer.

The term "NK cells" as used herein refers to CD3-negative/CD56-positive mononuclear cells, and have a cytotoxic activity against cells in which expression of MHC class I molecules is reduced or the expression is lost.

The term "hematopoietic precursor cells" as used herein includes any cells having differentiation potency into blood cells of any one of cell types. The hematopoietic precursor cells of the present invention include, but are not limited to, an umbilical cord blood, hematopoietic stem cells derived from an adult blood cell tissue such as a bone marrow, hematopoietic precursor cells differentiation induced from induced pluripotent stem cells, embryonic stem cells and/or adult stem cells, and hematopoietic precursor cells directly converted from differentiated cells of fibroblasts or the like. The hematopoietic precursor cells of the present invention are included in CD34-positive cells. The hematopoietic precursor cells of the present invention may be prepared, however, by a method using a marker other than CD34 as long as CD34-positive cells are substantially contained. The hematopoietic precursor cells of the present invention may be prepared by any procedures known to those skilled in the art. For example, in collecting mononuclear cells from an umbilical cord blood, specific gravity centrifugation may be employed. Besides, hematopoietic precursor cells present in an umbilical cord blood can be selectively collected from mononuclear cells derived from the umbilical cord blood by using immunomagnetic beads on which an antibody to a cell surface marker is immobilized. As the immunomagnetic beads, Dynabeads (registered trademark) manufactured by Dynal and available from Invitrogen, or CliniMACS (registered trademark) manufactured by Miltenyi Biotec may be used, but the immunomagnetic beads are not limited to these. On the immunomagnetic beads, an anti-CD34 antibody is preferably immobilized. However, immunomagnetic beads on which another specifically bonding partner such as an antibody to a cell surface marker different from CD34 is immobilized may be used as long as CD34-positive cells derived from the umbilical cord blood can be collected. Besides, the hematopoietic precursor cells can be isolated/identified by performing immunofluorescent staining with a specific antibody to a cell surface marker and by using a flow cytometer. In the expansion method of the present invention, mononuclear cells separated from an umbilical cord blood may be cryopreserved and thawed in accordance with a time of transplantation to a patient to be used for expanding NK cells in some cases. The cryopreservation and thaw of the cells may be performed any method known to those skilled in the art. For the cryopreservation of the cells, any of commercially available cell cryopreservation solutions is used in some cases.

If the hematopoietic precursor cells are differentiation induced from induced pluripotent stem cells, embryonic stem cells and/or adult stem cells, the hematopoietic precursor cells may be differentiation induced from undifferentiated pluripotent stem cells by employing a culturing condition not using feeder cells and a serum, such as one reported by Niwa, A. et al., (PLoS ONE 6(7): e22261 (2011)), in some cases. To be brief, human ES cells or human iPS cells are allowed to form colonies in a serum-free medium for retaining the cells in an undifferentiated state, the medium is replaced with a serum-free medium for differentiation induction supplemented with BMP4, and with this day set as day 0, the cells are cultured up to day 4. On day 4, the medium is replaced with a serum-free medium for differentiation induction supplemented with VEGF and SCF instead of BMP4, and the cells are cultured up to day 6. Thereafter, on day 6, the medium is replaced with a serum-free medium for differentiation induction supplemented with a stem cell factor (SCF), thrombopoietin (TPO), interleukin 3 (IL-3), FMS-like tyrosine kinase 3 ligand (Flt3L), a fusion protein of IL-6 receptor and IL-6 (FP6), and the like. On day 10 to 12, a cluster of hematopoietic cells starts to be observed in a margin of the colony, and starts to float in the medium several days later.

If the hematopoietic precursor cells are directly converted from differentiated cells such as fibroblasts, for example, a method reported by Szabo, E. et al. (Nature, 468: 521 (2010)) may be employed in some cases. To be brief, OCT4 protein is forcedly expressed in differentiated cells such as human fibroblasts, and cultured in a medium supplemented with a basic fibroblast growth factor (bFGF), insulin-like growth factor II (IGF-II), Flt-3L and SCF. After about 21 days, CD45-positive cells appear. The CD45-positive cells are transferred to another culture vessel, and are further cultured in a medium for hematopoietic differentiation supplemented with SCF, G-CSF, Flt-3L, IL-3, IL-6 and BMP-4. About a quarter of the CD45-positive cells obtained about 16 days after the cultivation in the medium for hematopoietic differentiation are positive also to CD34. Such cells are further differentiation induced into any of various blood cell types.

The term "umbilical cord blood" as used herein refers to both a fresh umbilical cord blood collected from an umbilical cord at the time of delivery and an umbilical cord blood in a frozen state available through an umbilical cord blood bank system in which an umbilical cord blood is cryopreserved after obtaining test data for histocompatibility.

The term "adult stem cells" as used herein refers to stem cells that are derived from a somatic cell tissue obtained from any one of an embryo after the period of organogenesis or a fetus, a placenta thereof, and an individual of any age after delivery, and that have differentiation potency into cells of at least one or more cell types. The term "induced pluripotent stem cells" as used herein refers to pluripotent stem cells induced from non-pluripotent cells such as those described by Takahashi K. and Yamanaka S. (Cell, 126, 663 (2006)), and can be induced by any induction method.

In the NK cells obtained by the preparation method of the present invention, the pharmaceutical composition containing the NK cells and the cell therapy of the present invention, a solution for suspending or culturing living cells is, for example, a saline, a phosphate buffered saline (PBS), a medium, a serum or the like in general. The solution may contain a carrier pharmaceutically acceptable as a pharmaceutical or a quasi-pharmaceutical in some cases.

The NK cells obtained by the preparation method of the present invention, the pharmaceutical composition containing the NK cells and the cell therapy of the present invention can be applied to treatment and/or prevention of various diseases having sensitivity to NK cells. Examples of such diseases include, but are not limited to, cancers and tumors such as an oral cancer, a gallbladder cancer, a cholangiocarcinoma, a lung cancer, a liver cancer, a colorectal cancer, a kidney cancer, a bladder cancer and leukemia, and infectious diseases caused by viruses, bacteria and the like. The pharmaceutical composition containing the NK cells of the present invention may contain, in addition to the NK cells prepared by the method of the present invention, an NK cell precursor, T cells, NKT cells, hematopoietic precursor cells and other cells in some cases. The cell therapy of the present invention may be practiced singly or in combination with surgical treatment, chemotherapy, radiation therapy or the like in some cases. In the cell therapy of the present invention, the NK cells expanded by the method of the present invention may be transplanted into a patient together with T cells and NKT cells in some cases. In the cell therapy of the present invention, the NK cells may be transplanted by, for example, intravenous, intraarterial, subcutaneous or intraperitoneal administration in some cases.

In the method for preparing NK cells of the present invention, in the method for preparing the pharmaceutical composition of the present invention and in the cell therapy of the present invention, any of media such as, but not limited to, a KBM501 medium (Kohjin Bio Co., Ltd.), a CellGro SCGM medium (registered trademark, Cellgenix, Iwai Chemicals Company), a STEMLINE II (Sigma-Aldrich Co. LLC.), an X-VIVO15 medium (Lonza, Takara Bio Inc.), IMDM, MEM, DMEM and RPMI-1640 may be singly used as or blended in an appropriate ratio to be used as a medium for culturing cells in some cases. Besides, the media for culturing cells may be used with supplementation of at least one additional component selected from the group consisting of a serum, a serum albumin, an appropriate protein, a cytokine, an antibody, a compound and another component, which will be described below, in some cases.

The medium may be supplemented with an autologous serum of a subject, a human AB-type serum available from BioWhittaker Inc. or the like, or a donated human serum albumin available from Japanese Red Cross Society in some cases. The autologous serum and the human AB-type serum is supplemented preferably in a concentration of 1 to 10%, and the donated human serum albumin is supplemented preferably in a concentration of 1 to 10%. The subject may be a healthy person, or a patient having any of various diseases sensitive to NK cells.

The medium may be supplemented with an appropriate protein, a cytokine, an antibody, a compound or another component as long as the effect of expanding NK cells is not impaired. The cytokine may be interleukin 2 (IL-2), interleukin 3 (IL-3), interleukin 7 (IL-7), interleukin 12 (IL-12), interleukin 15 (IL-15), interleukin 21 (IL-21), stem cell factor (SCF), thrombopoietin (TPO) and/or FMS-like tyrosine kinase 3 ligand (Flt3L) in some cases. The IL-2, IL-3, IL-7, IL-12, IL-15, IL-21, SCF, TPO and Flt3L preferably have a human amino acid sequence, and are preferably produced by a recombinant DNA technology from the safety viewpoint. The IL-15 is used preferably in a concentration of 0.1 to 100 ng/mL, more preferably in a concentration of 20 to 30 ng/mL, and particularly preferably in a concentration of 25 ng/mL. The SCF is used preferably in a concentration of 2 to 100 ng/mL, more preferably in a concentration of 20 to 30 ng/mL, and particularly preferably in a concentration of 25 ng/mL. The IL-7 is used preferably in a concentration of 0.5 to 100 ng/mL, more preferably in a concentration of 20 to 30 ng/mL, and particularly preferably in a concentration of 25 ng/mL. The Flt3L is used preferably in a concentration of 1 to 100 ng/mL, more preferably in a concentration of 20 to 30 ng/mL, and particularly preferably in a concentration of 25 ng/mL. The TPO is used preferably in a concentration of 1 to 100 ng/mL, more preferably in a concentration of 20 to 30 ng/mL, and particularly preferably in a concentration of 25 ng/mL.

Herein, the concentration of the IL-2 may be shown in Japanese Reference Unit (JRU) and International Unit (IU). Since 1 IU corresponds to approximately 0.622 JRU, 1750 JRU/mL corresponds to approximately 2813 IU/mL. The IL-2 preferably has a human amino acid sequence and is preferably produced by a recombinant DNA technology from the safety viewpoint. The IL-2 is used preferably in a concentration of 100 to 2900 IU/mL, more preferably in a concentration of 100 to 2813 IU/mL, and particularly preferably 2813 IU/mL.

In the preparation method of the present invention and in the cell therapy of the present invention, in the step of expanding hematopoietic precursor cells, the cells are cultured in a medium supplemented with IL-15, SCF, IL-7 and Flt3L. The medium may be supplemented further with TPO in some cases. The medium may be replaced at any time after starting the cultivation as long as a desired number of NK cells can be obtained, and is preferably replaced every 3 to 5 days. In the expansion of the hematopoietic precursor cells, the cell growth rate is abruptly lowered in about 5 weeks. Therefore, the expansion of the hematopoietic precursor cells is conducted for about 5 weeks, namely, for 32, 33, 34, 35, 36, 37 or 38 days, after starting the cultivation. Thereafter, from the expanded hematopoietic precursor cells, NK cells are differentiation induced. In the step of differentially inducing NK cells, the cells are cultured in a medium supplemented with IL-2. The differentiation induction of NK cells is conducted for about 1 week, namely, for 5, 6, 7, 8 or 9 days. Here, cultivation conducted for n days under a given culturing condition refers to that the cultivation is conducted from a cultivation start date to n days after under the culturing condition, and means that transition to a next culturing condition or cell collection is performed n days after starting the cultivation.

In the present invention, the hematopoietic precursor cells may be frozen during the expansion or after completing the expansion, and thawed in accordance with a time of transplantation into a patient to be used for the transplantation into the patient in some cases. The cells may be frozen and thawed by any of methods known to those skilled in the art. For freezing the cells, any of commercially available cryopreservation solutions is used in some cases.

In the expansion method of the present invention, the culture vessel includes, but is not limited to, commercially available dishes, flasks, plates and multi-well plates. The culturing condition is not especially limited as long as the effect of expanding NK cells is not impaired, but a culturing condition of 37°C, 5% CO₂ and a saturated water vapor atmosphere is generally employed. Since the purpose of the present invention is to prepare a large amount of NK cells, it is advantageous that the time period of culturing the cells in the medium is longer because a larger amount of NK cells can be thus obtained. The culture period is not especially limited as long as the NK cells can be expanded to a desired number of cells.

The method and the production of the pharmaceutical composition of the present invention are practiced preferably under conditions complying with good manufacturing practices (GMP) for pharmaceuticals and quasi-pharmaceuticals.

The cytotoxic activity of the NK cells thus prepared is evaluated by a method known to those skilled in the art. In general, the cytotoxic activity is quantitatively determined by incubating the NK cells (effector cells) and target cells labeled with a radioactive substance, a fluorescent dye or the like, and then measuring a radiation dose or a fluorescence intensity. The target cells may be K562 cells, acute myelogenous leukemia cells, or chronic myelogenous leukemia cells in some cases, but are not limited to these. The properties of the expanded NK cells may be checked by employing RT-PCR, solid phase hybridization, ELISA, Western blotting, immune precipitation, immunonephelometry, FACS, flow cytometry or the like in some cases.

In the present invention, the collection and cryopreservation of an umbilical cord blood and/or adult blood cell tissue, the preparation of an autologous serum, the preparation of an umbilical cord blood and/or adult blood cell tissue, and mononuclear cells differentiation induced from pluripotent stem cells such as induced pluripotent stem cells, embryonic stem cells or adult stem cells, the preparation of hematopoietic precursor cells from the mononuclear cells, the measurement of the number of cells before and after the cultivation of the hematopoietic precursor cells, the measurement of a constituent ratio among NK cells, T cells and other cell types in the hematopoietic precursor cells before and after the cultivation, the calculation of the expansion factor of the NK cells, and the statistical analysis of a measurement error or significance may be practiced by any methods known to those skilled in the art.

All the literatures mentioned herein are incorporated herein by reference.

### Brief Description of Drawings

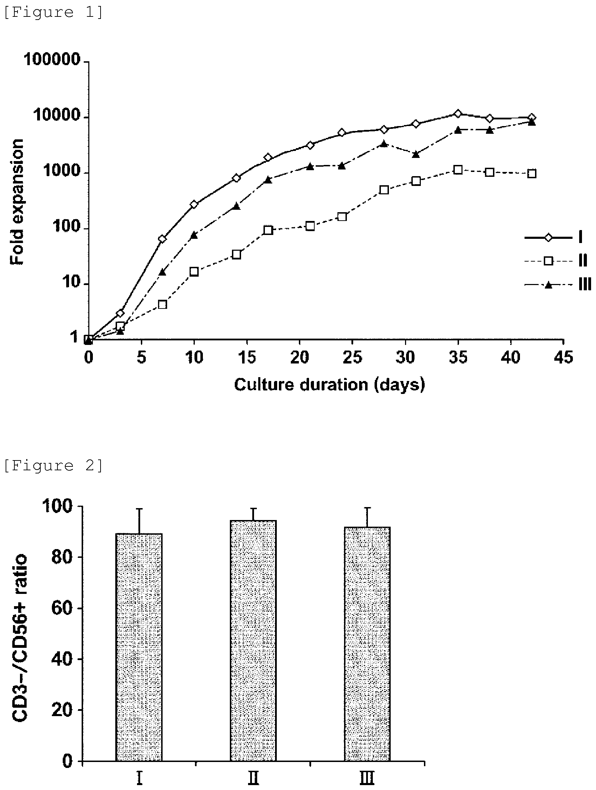

[Figure 1] Figure 1 illustrates line graphs for comparing change with time of the expansion factor of umbilical cord blood-derived hematopoietic precursor cells among a first protocol (I) in which the cells are expanded for 1 week by using an expansion culture medium 1, expanded for 4 weeks by using an expansion culture medium 2, and then cultured for 1 week with the medium replaced with a differentiation induction medium 1, a second protocol (II) in which the cells are expanded for 5 weeks by using the expansion culture medium 2 alone and then cultured for 1 week with the medium replaced with the differentiation induction medium 1, and a third protocol (III) in which the cells are expanded for 5 weeks by using an expansion culture medium 3 alone and then cultured for 1 week with the medium replaced with the differentiation induction medium 1.
[Figure 2] Figure 2 illustrates bar graphs of ratios of CD3-negative and CD56-positive NK cells obtained by the first protocol (I), the second protocol (II) and the third protocol (III).

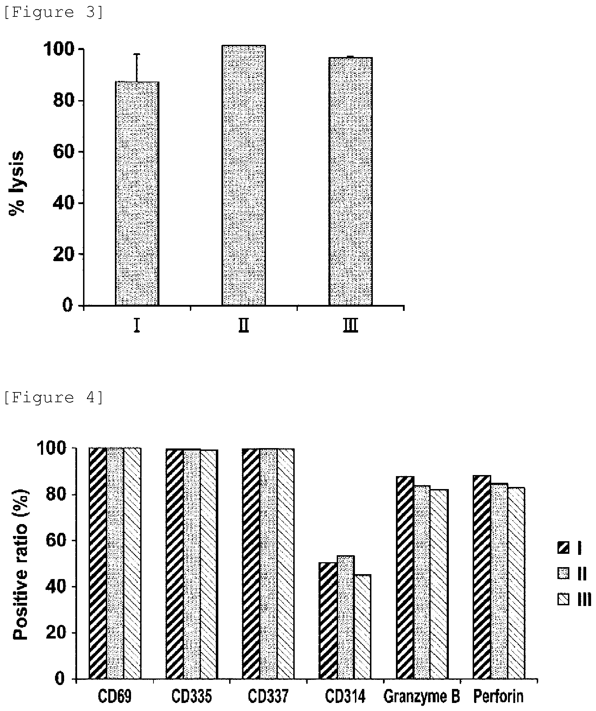

[Figure 3] Figure 3 illustrates bar graphs of cytotoxic activity, against K562 cells, of the NK cells obtained by the first protocol (I), the second protocol (II) and the third protocol (III).
[Figure 4] Figure 4 illustrates bar graphs of ratios, in the CD3-negative and CD56-positive NK cells, of cells positive to CD69, CD335 (NKp46), CD337 (NKp30), CD314 (NKG2D), granzyme B and perforin in the umbilical cord blood-derived hematopoietic precursor cells having been subjected to the expansion and differentiation induction in the respective protocols.

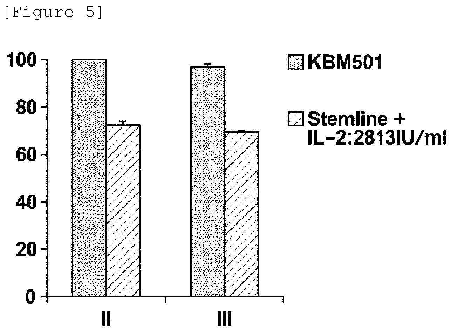

[Figure 5] Figure 5 illustrates bar graphs of cytotoxic activity, against K562 cells, of NK cells (KBM501) obtained by the second protocol (II) and the third protocol (III), and NK cells obtained when the differentiation induction media used in these protocols are replaced from KBM501 to a Stemline II medium supplemented with 1750 JRU/mL, namely, 2813 IU/mL, of IL-2 (Stemline + IL-2: 2813 IU/mL).

### Description of Embodiments

Examples of the present invention described below are intended to be merely illustrative, and do not limit the technical scope of the present invention. The technical scope of the present invention is defined merely by the appended claims. Modifications of the present invention, such as addition, deletion and replacement of a constituent feature of the present invention, can be conducted without departing from the spirit of the present invention.

### Example 1

This example was performed for proving that NK cells having high purity and high activity can be obtained from umbilical cord blood-derived hematopoietic precursor cells by an expansion method of the present invention.

### 1. Materials and Methods

### Human Umbilical Cord Blood-derived Hematopoietic Precursor Cells

A sample of human umbilical cord blood-derived hematopoietic precursor cells was obtained from PromoCell (Takara Bio Inc., C-12921) or ZenBio Inc. (B-Bridge Co., Ltd., SER-CD34-F). The sample was CD34-positive precursor cells purified from mononuclear cells by using immunomagnetic beads CD34 (CD34 positive rate: 90% or more) and frozen. It is noted that a written consent was obtained from the mother in collecting the sample. Besides, it was confirmed that the results of HIV virus test and hepatitis B virus test were negative.

### Media and Reagents

As a medium, STEMLINE II (Sigma-Aldrich Co. LLC., Catalog No. S0192, Lot No. SLBB3210) and/or a KBM501 medium (Kohjin Bio Co., Ltd., Catalog No. 16025015, Lot No. K1M120924, supplemented with 1750 JRU/mL, namely, 2813 IU/mL, of IL2 and 2000 mg/mL or less of a human serum albumin) was used, and was supplemented with a human AB-type serum (Kohjin Bio Co., Ltd., Catalog No. 12181301, Lot No. 12020165) at a final concentration of 10%. Proteins such as a cytokine used to add in experiments described below were all human recombinant proteins unless otherwise mentioned.

### Expansion (1) of Hematopoietic Precursor Cells

The umbilical cord blood-derived CD34-positive cells were thawed in accordance with the specifications of the manufacturer, and then diluted with an expansion culture medium 1 to a concentration of 5 x 10⁵ cells/mL, and seeded in a 6-well culture plate (140675, nunc, ThermoFisher Scientific). The medium was replaced on the 5th day of the cultivation. The expansion culture medium 1 was a STEMLINE II medium supplemented with 25 ng/mL of TPO, 25 ng/mL of SCF, 25 ng/mL of Flt3L, 250 pg/mL of G-CSF, 10 pg/mL of GM-CSF, 50 pg/mL of IL-6 and a 10% human AB-type serum.

### Expansion (2) of Hematopoietic Precursor Cells

The umbilical cord blood-derived CD34-positive cells were cultured in the expansion culture medium 1 for 1 week, washed with PBS three times, diluted with an expansion culture medium 2 to 5 x 10⁵ cells/mL, seeded in a 6-well culture plate (140675, nunc, ThermoFisher Scientific), and cultured for further 4 weeks. The medium was replaced every 4 days from the 3rd day of the cultivation. In replacing the medium, a cell suspension was collected and centrifuged at room temperature and 500 g for 5 minutes for removing the medium, and then, the resultant cells were diluted with a fresh expansion culture medium 2 to 5 x 10⁵ cells/mL and seeded in a 6-well culture plate. The expansion culture medium 2 was a STEMLINE II medium supplemented with 25 ng/mL of IL-15, 25 ng/mL of SCF, 25 ng/mL of IL-7, 25 ng/mL of Flt3L, and a 10% human AB-type serum.

### Expansion (3) of Hematopoietic Precursor Cells

The umbilical cord blood-derived CD34-positive cells were cultured for 5 weeks by using the expansion culture medium 2 alone. The medium was replaced every 4 days from the 3rd day of the cultivation.

### Expansion (4) of Hematopoietic Precursor Cells

The umbilical cord blood-derived CD34-positive cells were cultured for 5 weeks by using an expansion culture medium 3 alone. The medium was replaced every 4 days from the 3rd day of the cultivation. The expansion culture medium 3 was a STEMLINE II medium supplemented with 25 ng/mL of IL-15, 25 ng/mL of SCF, 25 ng/mL of IL-7, 25 ng/mL of Flt3L, 25 ng/mL of TPO and a 10% human AB-type serum.

### Differentiation Induction (1) into NK cells

The umbilical cord blood-derived CD34-positive cells were expanded by using any of the expansion culture media 1 to 3 for 35 days in total, and then, the medium was replaced with a differentiation induction medium 1 for inducing differentiation into NK cells for 7 days. The differentiation induction medium 1 was a KBM501 medium supplemented with a 10% human AB-type serum.

### Differentiation Induction (2) into NK Cells

The umbilical cord blood-derived CD34-positive cells were expanded by using the expansion culture medium 3 for 35 days in total, and then, the medium was replaced with a differentiation induction medium 2 for inducing differentiation into NK cells for 7 days. The differentiation induction medium 2 was a STEMLINE II medium supplemented with 1750 JRU/mL, namely, 2813 IU/mL, of IL-2 and a 10% human AB-type serum.

### Analysis of Number of Cells and Cell Surface Marker

The number of the hematopoietic precursor cells was determined by measuring the number of living cells by using a counting chamber. As cell surface markers of the cells, an anti-CD3 antibody (BioLegend Japan Inc., Catalog No. 317308), an anti-CD56 antibody (318321, BioLegend Japan Inc., Catalog No. 304607), an anti-CD69 antibody (BioLegend Japan Inc., Catalog No. 310905), an anti-CD335 (NKp46) antibody (BioLegend Japan Inc., Catalog No. 331907), an anti-CD337 (NKp30) antibody (BioLegend Japan Inc., Catalog No. 325207), an anti-CD314 (NKG2D) antibody (BioLegend Japan Inc., Catalog No. 320805), an anti-granzyme B antibody (BD Pharmingen, Catalog No. 560211, Japan Becton, Dickinson and Company) and an anti-perforin antibody (BioLegend Japan Inc., Catalog No. 308111) were used, and the analysis was performed by the flow cytometry.

### Cytotoxic Activity of Expanded NK Cells

The NK cells were expanded and differentiation induced in accordance with methods described in this example to be used as effector cells. K562 cells of chronic myelogenous leukemia cells were cultured by a method known to those skilled in the art to be used as target cells. The cytotoxic activity of the expanded NK cells and NK cells not expanded (hereinafter designated as the "non-expanded NK cells") was quantitatively determined by a method known to those skilled in the art. To be brief, the target cells were labeled by cultivation performed for 10 minutes in an RPMI-1640 medium supplemented with 3-3'-dioctadecyloxacarbocyanine (Sigma-Aldrich Co. LLC., Catalog No. D4292) in a final concentration of 0.01 mM. The target cells were washed with PBS(-) and an RPMI medium three times after labeling. The effector cells and the target cells were seeded in a round bottom 96-well culture plate, and cocultured in an RPMI medium for 4 hours. A ratio between the effector cells and the target cells (an E:T ratio) was adjusted to 2:1. The cytotoxic activity (%) was quantitatively determined by the flow cytometry by using 7-amino-actinomycin D (Sigma-Aldrich Co. LLC., A9400).

### 2. Results

### Expansion of Umbilical Cord Blood-derived Hematopoietic Precursor Cells

Figure 1 illustrates line graphs for comparing change with time of the expansion factor of the umbilical cord blood-derived hematopoietic precursor cells among the first protocol (corresponding to a graph plotted with white rhombuses (◊), I) in which the cells were expanded for 1 week by using the expansion culture medium 1, expanded for 4 weeks by using the expansion culture medium 2, and then cultured for 1 week with the medium replaced with the differentiation induction medium 1, the second protocol (corresponding to a graph plotted with white squares (□), II) in which the cells were expanded for 5 weeks by using the expansion culture medium 2 alone and then cultured for 1 week with the medium replaced with the differentiation induction medium 1, and the third protocol (corresponding to a graph plotted with black triangles (▲), III) in which the cells were expanded for 5 weeks by using the expansion culture medium 3 alone and then cultured for 1 week with the medium replaced with the differentiation induction medium 1. The abscissa indicates the number of days from the start of the cultivation of the hematopoietic precursor cells prepared from the umbilical cord blood. The ordinate indicates the expansion factor obtained on the assumption that the number of cells at the start of the cultivation was 1. As illustrated in Figure 1, the growth rate of the umbilical cord blood-derived hematopoietic precursor cells was largely lowered from about the 30th day of the cultivation. While the expansion factors of the cells obtained by the first and third protocols were both approximately 10,000, the expansion factor of the cells obtained by the second protocol was approximately 1,000, and thus the number of the cells was increased by merely 1/10 as compared with that obtained by the first and third protocols. Incidentally, even when the cells were cultured in the expansion culture medium up to day 42, the number of cells was not substantially changed as compared with that obtained on day 35 (not shown).

Figure 2 illustrates bar graphs of ratios of CD3-negative and CD56-positive NK cells contained in the umbilical cord blood-derived hematopoietic precursor cells having been subjected to the expansion and differentiation induction by the first protocol (I), the second protocol (II) and the third protocol (III). As illustrated in Figure 2, while the ratio of the NK cells was approximately 90% in the cells expanded and differentiation induced by the second protocol, the ratio of the NK cells was merely approximately 80% in the cells expanded and differentiation induced by the first and third protocols. As illustrated in Figure 1, however, the expansion factor of the cells obtained by the second protocol was as low as 1/10 of the expansion factor of the cells obtained by the first and third protocols. Therefore, it was proved that the cells obtained by the third protocol contain a larger number of NK cells.

Figure 3 illustrates bar graphs of the cytotoxic activity, against the K562 cells, of the NK cells obtained by the first protocol (I), the second protocol (II) and the third protocol (III). The ordinate indicates a percentage of the target cells, K562 cells, lysed when the cells were mixed in the ratio between the effector cells and the target cells (E:T ratio) of 2:1 and cocultured for 4 hours. Here, the umbilical cord blood-derived hematopoietic precursor cells having been subjected to the expansion and the differentiation induction by the respective protocols were directly used as the effector cells without further purifying the NK cells. As illustrated in Figure 3, the cell lysis ratio of the NK cells obtained by the second protocol was approximately 100%, and the cytotoxic activity was extremely high, but as for the cytotoxic activity of the NK cells obtained by the third protocol, the cell lysis ratio was approximately 95%. As illustrated in Figure 1, the expansion factor of the cells obtained by the second protocol was as low as 1/10 of the expansion factor of the cells obtained by the first and third protocols. Therefore, it was found that the cells obtained by the third protocol contain a large number of cells having a higher cytotoxic activity than the cells obtained by the second protocol. According to the report of Non Patent Literature 3, the cytotoxic activity targeting the K562 cells is merely approximately 40% under the same coculturing condition. Accordingly, it was proved that the NK cells obtained by the method of the present invention have much higher cytotoxic activity than umbilical cord blood-derived NK cells obtained by the conventional technique although the expansion factor is substantially equivalent.

Figure 4 illustrates bar graphs of ratios, in the CD3-negative and CD56-positive NK cells, of cells positive to CD69, CD335 (NKp46), CD337 (NKp30), CD314 (NKG2D), granzyme B and perforin in the umbilical cord blood-derived hematopoietic precursor cells having been subjected to the expansion and the differentiation induction by the respective protocols. In all the NK cells obtained through the expansion and the differentiation induction performed by any of the protocols, CD69, CD335 and CD337 were expressed in substantially 100% of the cells. A ratio of cells expressing CD314 (NKG2D) and cells expressing granzyme B or perforin was not largely different among all the NK cells obtained through the expansion and the differentiation induction performed by any of the protocols.

Figure 5 illustrates bar graphs of the cytotoxic activity, against K562 cells, of NK cells (KBM501) obtained by the second protocol (II) and the third protocol (III), and NK cells obtained when the differentiation induction media used in the respective protocols were replaced from the KBM501 to a Stemline II medium supplemented with 2813 IU/mL of IL-2 (Stemline + IL-2: 2813 IU/mL). When the differentiation induction medium was replaced from the KBM501 to the Stemline II medium supplemented with 2813 IU/mL of IL-2, the cytotoxic activity was lowered to 70%, but this activity was much higher than the cytotoxic activity reported in Non Patent Literature 3 (approximately 40%). Accordingly, it was proved that the effects of the present invention can be exhibited if a differentiation induction medium supplemented with IL-2 is used regardless of the composition of a basal medium.

## Claims

1. A method for preparing NK cells, comprising the steps of expanding hematopoietic precursor cells under a single culturing condition using a medium supplemented with IL-15, SCF, IL-7 and Flt3L; and
differentially inducing the cells obtained in the expanding step into NK cells for 5 to 9 days under a culturing condition using a medium supplemented with 2500 IU/ml to 2900 IU/ml of IL-2.

2. The method of claim 1, wherein in the expanding step the medium is supplemented with 2813 IU/ml to 2900 IU/ml of IL-2.

3. The method for preparing NK cells according to claim 1 or 2, wherein the medium used in the step of expanding hematopoietic precursor cells under a single culturing condition is further supplemented with TPO.

4. The method for preparing NK cells according to any one of claims 1 to 3, wherein the medium used in each of the steps is supplemented with a human AB-type serum and/or a human serum albumin.

5. The method for preparing NK cells according to any one of claims 1 to 4, wherein the hematopoietic precursor cells are at least one of hematopoietic precursor cells selected from the group consisting of hematopoietic precursor cells contained in an umbilical cord blood and/or an adult blood cell tissue, hematopoietic precursor cells differentially induced from induced pluripotent stem cells, embryonic stem cells and/or adult stem cells, and hematopoietic precursor cells directly converted from differentiated cells.

6. A method for preparing a pharmaceutical composition comprising NK cells for use in cell therapy, wherein the method comprises the steps of expanding hematopoietic precursor cells under a single culturing condition using a medium supplemented with IL-15, SCF, IL-7 and Flt3L; and
differentially inducing the cells obtained in the expanding step into NK cells for 5 to 9 days under a culturing condition using a medium supplemented with 2500 IU/ml to 2900 IU/ml of IL-2.

7. A method according to claim 6, wherein the pharmaceutical composition further comprises one or more of an NK cell precursor, T cells, an NKT cell and hematopoietic precursor cells.

8. A method according to claim 6 or 7, wherein the method further comprises suspending the NK cells in a solution comprising a pharmaceutical acceptable carrier.

## Patentansprüche

1. Verfahren zum Herstellen von NK-Zellen, umfassend die Schritte des Expandierens hämatopoetischer Vorläuferzellen unter einer einzigen Bedingung des In-Kultur-Nehmens unter Verwendung eines Mediums, das mit IL-15, SCF, IL-7 und Flt3L ergänzt ist; und
differentielles Induzieren der Zellen zu NK-Zellen, die in dem Schritt des Expandierens für 5 bis 9 Tage unter einer Bedingung des In-Kultur-Nehmens erhalten wurden, indem ein Medium verwendet wird, das mit 2.500 IE/ml bis 2.900 IE/ml von IL-2 ergänzt ist.

2. Verfahren nach Anspruch 1, wobei das Medium in dem Schritt des Expandierens mit 2.813 IE/ml bis 2.900 IE/ml von IL-2 ergänzt wird.

3. Verfahren zum Herstellen von NK-Zellen nach Anspruch 1 oder 2, wobei das Medium, das in dem Schritt des Expandierens hämatopoetischer Vorläuferzellen unter einer einzigen Bedingung verwendet wird, ferner mit TPO ergänzt wird.

4. Verfahren zum Herstellen von NK-Zellen nach einem der Ansprüche 1 bis 3, wobei das Medium, das in jedem der Schritte verwendet wird, mit einem menschlichen Serum vom AB-Typ oder menschlichen Albumin-Serum ergänzt wird.

5. Verfahren zum Herstellen von NK-Zellen nach einem der Ansprüche 1 bis 4, wobei die hämatopoetischen Vorläuferzellen mindestens eine der hämatopoetischen Vorläuferzellen sind, die ausgewählt sind aus der Gruppe bestehend aus hämatopoetischen Vorläuferzellen, die in einem Nabelschnurblut und/oder einem adulten Blut-Zellgewebe enthalten sind, hämatopoetischen Vorläuferzellen, die differentiell aus induzierten pluripotenten Stammzellen induziert sind, embryonalen Stammzellen und/oder adulten Stammzellen, und hämatopoetischen Vorläuferzellen, die direkt aus differenzierten Zellen umgewandelt worden sind.

6. Verfahren zum Herstellen einer pharmazeutischen Zusammensetzung, die NK-Zellen zur Verwendung in der Zelltherapie umfassen, wobei das Verfahren die Schritte des Expandierens hämatopoetischer Vorläuferzellen unter einer einzigen Bedingung des In-Kultur-Nehmens umfasst, indem ein Medium verwendet wird, das mit IL-15, SCF, IL-7 und Flt3L ergänzt ist; und
differentielles Induzieren der Zellen zu NK-Zellen, die in dem Schritt des Expandierens für 5 bis 9 Tage unter einer Bedingung des In-Kultur-Nehmens erhalten wurden, indem ein Medium verwendet wird, das mit 2.500 IE/ml bis 2.900 IE/ml von IL-2 ergänzt ist.

7. Verfahren nach Anspruch 6, wobei die pharmazeutische Zusammensetzung ferner eine oder mehrere eines NK-Zellen-Vorläufers, T-Zellen, eine NKT-Zelle und hämatopoetische Vorläuferzellen umfasst.

8. Verfahren nach Anspruch 6 oder 7, wobei das Verfahren ferner Suspendieren der NK-Zellen in einer Lösung umfasst, die einen pharmazeutisch verträglichen Träger umfasst.

## Revendications

1. Procédé pour préparer des cellules NK, comprenant les étapes consistant à expanser des cellules précurseurs hématopoïétiques dans une condition de culture unique en utilisant un milieu complété avec IL-15, SCF, IL-7 et Flt3L; et
à induire différentiellement les cellules obtenues dans l'étape d'expansion en cellules NK pendant 5 à 9 jours dans une condition de culture utilisant un milieu complété avec 2500 UI/ml à 2900 UI/ml de IL-2.

2. Procédé selon la revendication 1, dans lequel dans l'étape d'expansion le milieu est complété avec 2813 UI/ml à 2900 UI/ml de IL-2.

3. Procédé pour préparer des cellules NK selon la revendication 1 ou 2, dans lequel le milieu utilisé dans l'étape d'expansion de cellules précurseurs hématopoïétiques dans une condition de culture unique est en outre complété avec TPO.

4. Procédé pour préparer des cellules NK selon l'une quelconque des revendications 1 à 3, dans lequel le milieu utilisé dans chacune des étapes est complété avec un sérum de type AB humain et/ou de la sérumalbumine humaine.

5. Procédé pour préparer des cellules NK selon l'une quelconque des revendications 1 à 4, dans lequel les cellules précurseurs hématopoïétiques sont au moins l'une parmi les cellules précurseurs hématopoïétiques choisies dans le groupe constitué par les cellules précurseurs hématopoïétiques contenues dans le sang d'un cordon ombilical et/ou un tissu à cellules sanguines adultes, les cellules précurseurs hématopoïétiques induites différentiellement à partir de cellules souches pluripotentes induites, les cellules souches embryonnaires et/ou les cellules souches adultes, et les cellules précurseurs hématopoïétiques directement converties à partir de cellules différentiées.

6. Procédé pour préparer une composition pharmaceutique comprenant des cellules NK pour l'utilisation en thérapie cellulaire, lequel procédé comprend les étapes consistant à expanser des cellules précurseurs hématopoïétiques dans une condition de culture unique en utilisant un milieu complété avec IL-15, SCF, IL-7 et Flt3L; et
à induire différentiellement les cellules obtenues dans l'étape d'expansion en cellules NK pendant 5 à 9 jours dans une condition de culture utilisant un milieu complété avec 2500 UI/ml à 2900 UI/ml de IL-2.

7. Procédé selon la revendication 6, dans lequel la composition pharmaceutique comprend en outre un ou plusieurs parmi un précurseur de cellules NK, les lymphocytes T, une cellule NKT et les cellules précurseurs hématopoïétiques.

8. Procédé selon la revendication 6 ou 7, lequel procédé comprend en outre la mise en suspension des cellules NK dans une solution comprenant un véhicule pharmaceutique acceptable.
